# EUROPEAN PATENT SPECIFICATION

(11) **EP 1 741 717 B1**
(45) Date of publication and mention of the grant of the patent: **28.01.2009**
(21) Application number: 06013913.6
(22) Date of filing: 05.07.2006
(51) Int. Cl.: C07F 1/00, A61K 31/28

(54) **Metal nanostructures and pharmaceutical compositions**
Metallnanostrukturen und pharmazeutische Zusammensetzungen
Nanostructures métalliques et compositions pharmaceutiques

(30) Priority: 05.07.2005 US 696576 P
(43) Date of publication of application: 10.01.2007
(73) Proprietor: Interuniversitair Microelektronica Centrum, 3001 Leuven (BE)
(72) Inventor: Frederix, Filip, B-3001 Leuven (BE); Van de Broek, Bieke, B-3600 Genk (BE)
(74) Representative: Bird, William Edward

(56) References cited:
- TAKAE, SEIJI ET AL: "Ligand density effect on biorecognition by PEGylated gold nanoparticles : regulated interaction of RCA120 lectin with lactose installed to the distal end of tethered PEG strands on gold surface" BIOMACROMOLECULES , 6(2), 818-824 CODEN: BOMAF6; ISSN: 1525-7797, 2005, XP002402846
- PEELEN, DORA ET AL: "Immobilization of Amine-Modified Oligonucleotides on Aldehyde-Terminated Alkanethiol Monolayers on Gold" LANGMUIR , 21(1), 266-271 CODEN: LANGD5; ISSN: 0743-7463, 2005, XP002402847

## Description

### Field of invention

The present invention relates to metal nanostructures having a stable shape and/or reactive groups on their surface. The present invention also relates to metal nanostructures linked to biomolecules. The present invention also relates to a method to prepare metal nanostructures. The present invention also relates the use of metal nanostructures in the preparation of pharmaceutical composition active under infra-red (IR) radiation. The present invention also relates to a film consisting of metal nanostructures and to the use of this film for measuring a change of refractive index.

### Background of the invention

Shape has a strong influence on the physical properties of metal nanostructures. For instance, gold nanostructures of different shapes behave differently in presence of electromagnetic waves such as IR, visible or ultraviolet (UV) irradiation. In sensing, plasmonic or hyperthermia applications, nanostructures having non-spherical shapes perform usually better than their spherical counterparts. In thermotherapy, i.e. in heat treatment, the temperature of the tissue is elevated artificially with the aim of gaining therapeutic benefits. Thermotherapy is considered an adjunct to other treatments. One problem with treating cancer successfully is the fact that cancerous cells are very difficult to target specifically. In most respects, they are like normal cells, and even if they are not, they can hide the differences. But malignant cells are reliably more sensitive to heat than normal cells. Raising the temperature of the tumour is one way to selectively destroy cancer cells.

Thermotherapy exerts its beneficial effect in several ways, according to the current understanding. Several studies have shown increased apoptosis in response to heat. Hyperthermia damages the membranes, cytoskeleton, and nucleus functions of malignant cells. It causes irreversible damage to cellular perspiration of these cells. Heat above 41 °C also pushes cancer cells toward acidosis (decreased cellular pH) which decreases the viability of the cells and their transplant ability. Tumours have a tortuous growth of vessels providing them blood, and these vessels are unable to dilate and dissipate heat as normal vessels do. So tumours take longer to heat up, but then concentrate the heat within themselves. Tumour blood flow is increased by hyperthermia despite the fact that tumour-formed vessels do not expand in response to heat. Normal vessels are incorporated into the growing tumour mass and are able to dilate in response to heat, and to channel more blood into the tumour.

Tumour masses tend to have hypoxic (oxygen deprived) cells within the inner part of the tumor. These cells are resistant to radiation, but they are very sensitive to heat. This is why hyperthermia is an ideal companion to radiation: radiation kills the oxygenated outer cells, while hyperthermia acts on the inner low-oxygen cells, oxygenating them and so making them more susceptible to radiation damage. It is also thought that hyperthermia's induced accumulation of proteins inhibits the malignant cells from repairing the damage sustained.

All these properties make from thermotherapy a promising technique to combat cancer. Since the shape of metal nanostructures influences their response to infra-red radiation, shape control of metal nanostructures for use in thermotherapy is important.

The exact nucleation mechanism involved in the synthesis of metal nanostructures remains a mystery due to the length and the time scales on which it occurs and the variety of systems in which it may take place. Growth of the nucleus into larger nanocrystallites (i.e. seeds) likely occurs through a combination of aggregation and atomic addition.

Various strategies have been followed to control the shape of nanostructures. They either involve varying the type of seeding method employed or the type of inorganic ions introduced in the crystalisation media, the use of templates, the use of light or the use of capping agents. Facet-selective capping agents promote the abundance of a particular shape by selectively interacting with a specific crystallographic facet via chemical adsorption.

Some of those methods have been proved successful and cubes, triangular nanoplates, pyramids, branched and rod-like nanostructures have been produced. Although those special morphologies are promising in a variety of applications, they are currently not applied in real technologies. One of the major reasons is the stability of these nanostructures.

Hao et al. (Nanoletters 2004, vol. 4, n° 2, 327-330), for example, describes the synthesis and optical properties of branched gold nanocrystals. Those nanostructures can be kept for several days in a refrigerator, although eventually a spectral shift toward shorter wavelengths takes place, indicative of nanostructure annealing. This is indicative that the nanostructures loose their branched morphology and relax into spherical nanostructures.

Aside from the stability problem, it is often desirable that nanostructures contain functional groups which allow the nanostructures to covalently or electrostatically bind to biomolecules, surfaces or other materials. For instance, Chen et al (Nanoletters 2005, Vol. 5, n° 3, 473-477) described the functionalisation of gold hollow nanocages with compounds of the following formula:

IgG-HN-CO-CH₂-CH₂-(O-CH₂-CH₂)₈-S-S-(CH₂-CH₂-O)₈-CH₂-CH₂-CO-NH-IgG,

wherein IgG is an antibody (anti-mouse immunoglobulin G). The aim of this functionalisation is to target cancer cells. Although this approach permits to introduce functionalities on the nanostructure, Chen et al is silent on the issue of shape stability.

Takae, Seiji et al. (Biomacromolecules, 6(2), 818-824 discloses PEGylated gold nanoparticles and their dispersion stability is discussed. The problem of shape stability is not discussed in this article.

There is therefore a need in the art for a method to stabilise the shape of nanostructures. In particular, there is a need in the art for a method which permits to simultaneously stabilise the shape of nanostructures and introduce functionalities on the surfaces of nanostructures.

### Summary of the invention

An object of the present invention is to provide metal nanostructures, especially non-spherical metal nanostructures with an improved shape stability. Another object of the present invention is to provide a method, in particular a water-based method in order to prepare these metal nanostructures.

Broadly speaking, the invention is based on the unexpected finding that the shape of nanometric metal cores can be stabilized by contacting these nanometric metal cores with one or more molecules of the general formula W'-X-Y-Z or Z-Y-X-W-W-X-Y-Z, wherein W and W' are atoms or chemical groups able to bind to said nanometric metal core, X is a hydrophobic spacer, Y is a hydrophilic spacer and Z is either hydrogen or a reactive group able to bind a reactive substrate or biomolecule.

In a first embodiment, the present invention relates to a metal nanostructure comprising:
- a nanometric metal core comprising gold, silver or an assembly or alloy of gold and silver, and
- one or more molecules attached to one or more surfaces of the nanometric metal core,
wherein said molecules have the general formula W-X-Y-Z, wherein W is an atom selected from sulfur and selenium bound to said nanometric metal core, X is a hydrophobic spacer, preferably a hydrocarbon chain having from 8 to 30 carbon atoms, Y is a hydrophilic spacer and Z is either hydrogen or is selected from the group consisting of amines, thiols, carboxylic acid halides, carboxylic acid anhydrides, isocyanates, isothiocyanates, sulfonyl halides, aldehydes, imines, epoxides, ketones, phosphoric esters, alcohols and the following species:

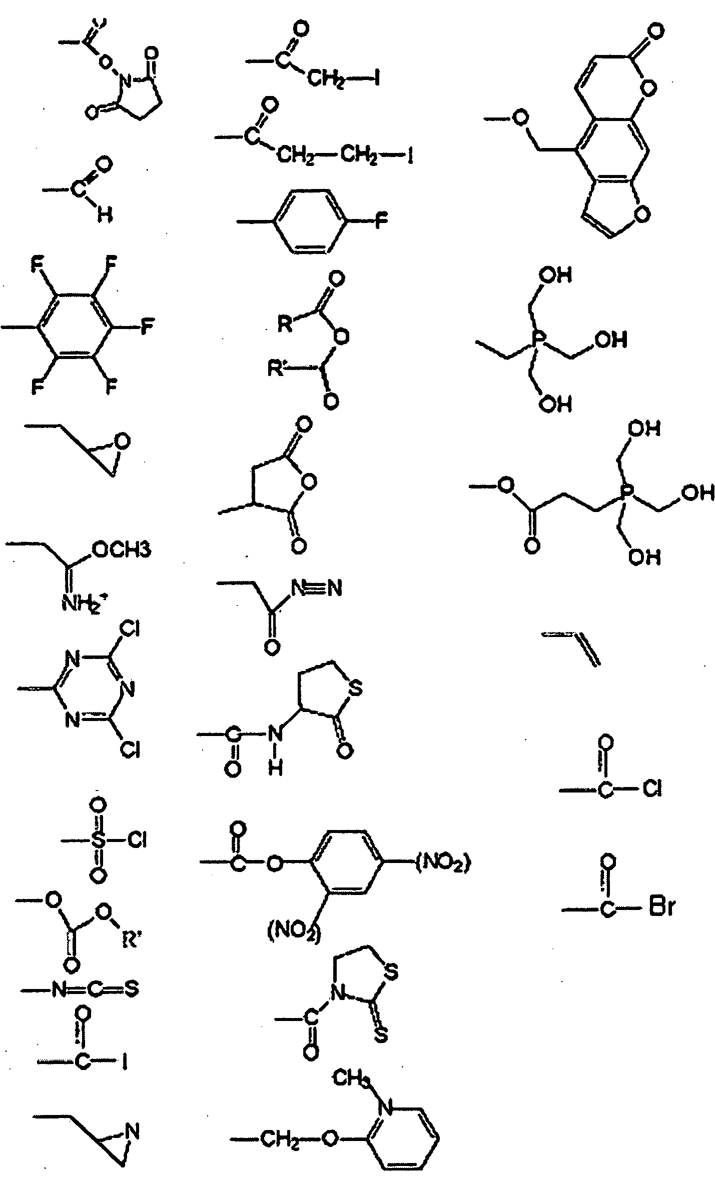

This embodiment is advantageous because the hydrophilic spacer Y permits a water-based preparation of the metal nanostructure and improves the water-suspendability of the metal nanostructures while the hydrophobic spacer X ensures the stability of a layer, preferably a monolayer, that the molecule W-X-Y-Z may form at one or more surfaces of the nanometric metal core.

As an additional feature, at least one of the one or more molecules may have a Z reactive group able to bind, preferably covalently or electrostatically, a reactive substrate or a biomolecule. As an additional sub-feature of the above described additional feature, the metal nanostructure may have one or more reactive group Z attached to a biomolecule. This feature is advantageous in medical applications because it permits to target the metal nanostructure to a specific location in a mammalian body for which the biomolecule presents a particular affinity.

As an additional feature, the nanometric metal core may have each of its dimensions from about 1 to 100 nm. This feature is advantageous in medical applications because its small size permits it to enter cancer cells and confer particular electromagnetic properties to the metal nanostructures.As an additional feature, the metal nanostructure of the present invention may have a pyramidal or a branched shape. This feature is advantageous because those shapes permits the production of much heat under IR irradiation, when compared to metal nanostructures of spherical shape.

As an additional feature according to the present invention, one or more, preferably each, of the W-X-Y-Z molecules may form a monolayer at one or more surfaces of the nanometric metal core. This feature is advantageous, because it enables to cost-efficiently cover the whole surface of the nanometric metal core, thereby stabilising it.

As an additional feature, W may be an atom selected from sulfur and selenium. This feature is advantageous because sulfur and selenium atoms have a high affinity for gold and/or silver and/or their alloys.

As an additional feature, Y may be a hydrophilic spacer that contains hydrogen-bond acceptors, do not contain hydrogen bond donors and is overall electrically neutral. This feature is advantageous because these properties are favourable to avoid non-specific binding to biomolecules, cells or tissues.

As an additional feature, Y may be a polyethylene glycol, a monosaccharide, an oligosaccharide, a polysaccharide, a N-acetylpiperazine, a permethylated sorbitol group or an oligosarcosine. This feature is advantageous because in medical applications these hydrophilic linkers are biocompatible and have also the property to avoid non-specific binding to biomolecules, cells or tissues.

In another embodiment, the present invention relates to a pharmaceutical composition including a metal nanostructure as described above and optionally one or more pharmaceutically acceptable excipients. The proportion of metal nanostructures in this composition may be from about 0.01 to 99.99 % by weight, preferably from about 0.05 to about 20% by weight, more preferably from about 0.1 to about 10% by weight. This is advantageous because pharmaceutical composition including the metal nanostructures of the present invention can be used in medical applications such as, but not limited to, the hyperthermic treatment of cancer.

As an additional feature, this pharmaceutical composition is active under infrared radiation, preferably at a wavelength between about 750 and about 1400 nm.

These is advantageous because at these wavelengths the metal nanostructures of the present invention are the most responsive.

In another embodiment, the present invention relates to an exemplary, but non-limiting, process for preparing a metal nanostructure as described in an embodiment above, characterised in that a nanometric metal core is contacted with one or more molecules having the general formula W'-X-Y-Z or Z-Y-X-W-W-X-Y-Z, wherein each of W and W' is an atom selected fro sulfur and selenium, X is a hydrophobic spacer, Y is a hydrophilic spacer and Z is either hydrogen or is selected from the group consisting of amines, thiols, carboxylic acid halides, carboxylic acid anhydrides, isocyanates, isothiocyanates, sulfonyl halides, aldehydes, imines, epoxides, ketones, phosphoric esters, alcohols and the following species:

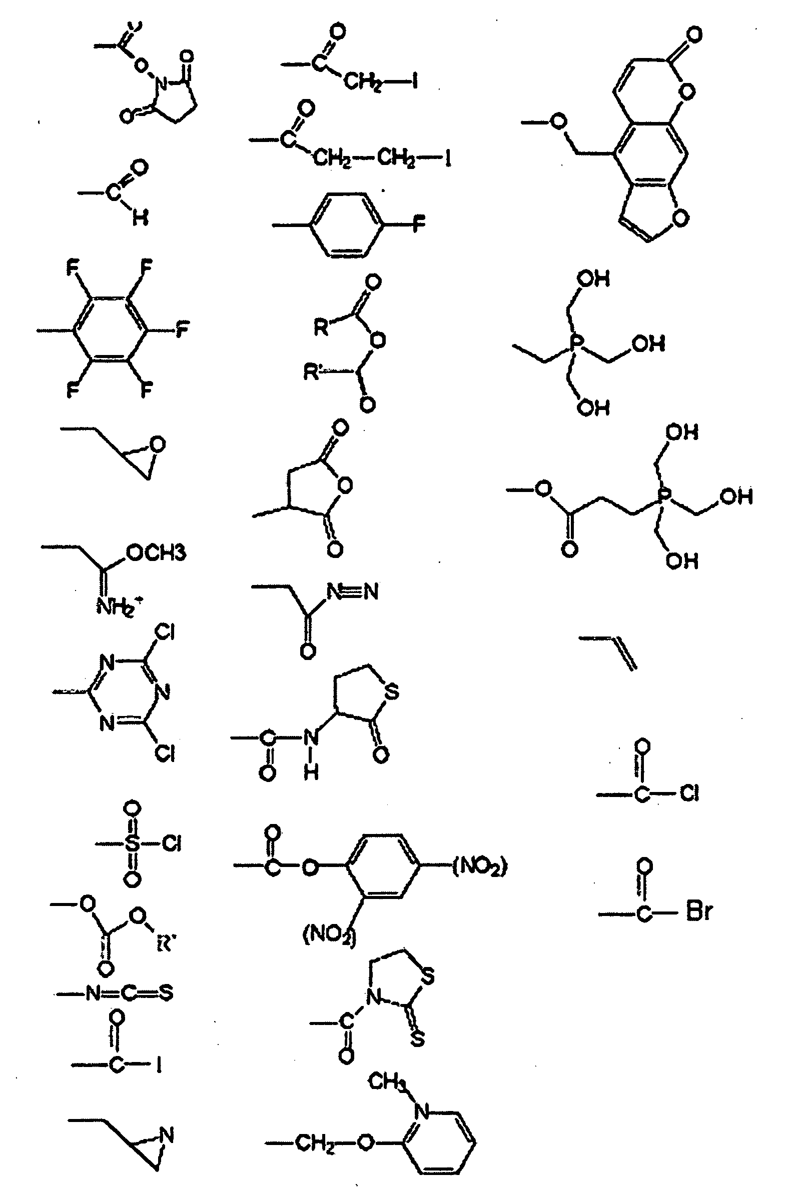

As an additional feature, one or more capping agents may be attached to said nanometric metal core and the preparation of the metal nanostructures then operates by exchanging these capping agents with one or more molecules having the general formula W'-X-Y-Z or Z-Y-X-W-W-X-Y-Z. This feature is advantageous because it enables to stabilise the non-spherical shapes obtainable when the nanometric metal core is synthesised in presence of capping agents. In the absence of such capping agents, shape control may often be inefficient and/or very limited in extent.

As an additional feature, the one or more molecules having the general formula W'-X-Y-Z or Z-Y-X-W-W-X-Y-Z may be water soluble. This is advantageous because it permits the preparation method of the metal nanostructures of this invention to be water-based.

As an additional feature, the capping agents may be selected from the group consisting of CH ₃ (CH₂)ₙN⁺(CH₃)₃ X⁻ (wherein n = integer of 1 to 15 and X = Br or Cl), (CH₃ (CH₂)ₙ)₄N⁺ X⁻ (wherein n = integer of 1 to 15, and X = Br or Cl), benzyldimethylammonium chloride (BDAC), bis(p-sulfonatophenyl) phenylphosphine (BSPP), bis(2-ethylhexyl)sulfosuccinate (AOT), octylamine, dimyristoyl-*L*-α-phosphatidyl-*DL*-glycerol (DMPG), sodium dodecylsulfonate (SDS), ascorbic acid and sodium citrate. This is advantageous because these capping agents are readily available and known to efficiently induce specific shapes to metal nanostructures containing gold or silver.

As an additional feature, W' may be a chemical group selected from R-S- and R-Se-, wherein R is selected from the group consisting of hydrogen and C₁-C₃₀ alkyl (preferably C₁-C₄ alkyl), and W may be an atom selected from sulfur and selenium.

In another embodiment, the present invention relates to a film comprising, preferentially consisting of, metal nanostructures according to any embodiment of the present invention as described herein.

In another embodiment the present invention relates to a process for making a film comprising the steps of:
(i) suspending metal nanostructures in an aqueous medium, and
(ii) depositing the resulting suspension onto a substrate.

In another embodiment, the present invention relates to the use of a film comprising metal nanostructures according to an embodiment of the present invention for measuring a change of refractive index.

More specifically, the present invention relates to a method for measuring a change of refractive index comprising the steps of:
(i) forming a film comprising metal nanostructures according to any embodiment of the present invention as described herein,
(ii) measuring the wavelength of maximal absorption of said film in a medium having a first refractive index,
(iii) measuring the wavelength of maximal absorption of said film in a medium having a second refractive index,
(iv) calculating the difference between the wavelength of maximal absorption measured in (ii) and the wavelength of maximal absorption measured in (iii), and
(v) relating this calculated difference to a change in refractive index.

In another embodiment, the present invention relates to a method of therapy of a patient, preferably a mammal such as a human being, having cancer tumour, comprising the steps of:
- injecting metal nanostructures according to any embodiment of the present invention into a tumour, and
- exposing the tumour to infrared irradiation, preferably at a wavelength between about 750 and about 1400 nm.

In another embodiment, the present invention relates to a method of therapy a patient, preferably a mammal such as a human being, having cancer tumour, comprising the step of injecting into the bloodstream of said patient a metal nanostructure attached to a biomolecule, said biomolecule having a particular affinity for cancer cells, and exposing the tumour to infrared irradiation, wherein the metal nanostructures are according to any embodiment of the present invention.

### Brief description of the drawings

Figure 1 is a schematic representation of a process for preparing metal nanostructures according to an embodiment of the present invention.
Figure 2 is a schematic representation of a process for reacting biomolecules to nanometric metal cores according to an embodiment of the present invention.
Figure 3 shows the dependence of the oprical properties of branched nanometric gold cores toward the addition rate of HAuCl₄.
Figure 4 shows the influence of the age of the mixture citrate/BSPP on the band positions of absorption maxima of branched nanometric gold cores.
Figure 5 shows the influence of the HAuCl₄ concentration on the band positions of absorption maxima of branched nanometric gold cores.
Figure 6 shows the influence of the amount of BSPP on the band positions of absorption maxima and band width of branched nanometric gold cores.
Figure 7 shows the influence of the amount of H₂O₂ on the band positions of absorption maxima and band width of branched nanometric gold cores.
Figure 8 shows the UV-Visible spectra of branched nanometric gold cores plated with various amounts of silver in solution.
Figure 9 shows the UV-Visible spectra of branched nanometric gold cores plated with silver on a surface.
Figure 10 shows the UV-Visible spectra of branched metal nanostructures according to an embodiment of the present invention.

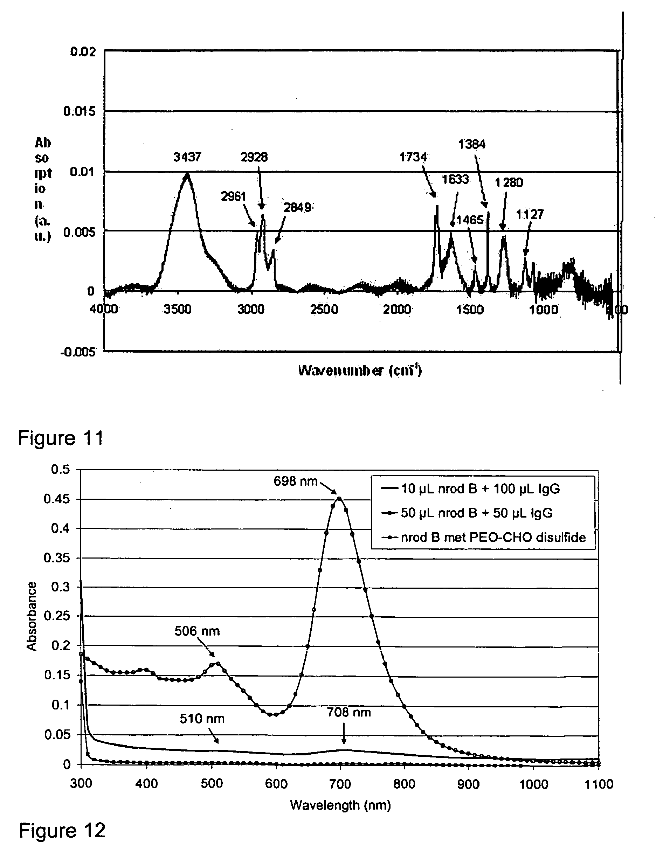

Figure 11 is the FTIR spectrum of metal nanostructures according to an embodiment of the present invention.
Figure 12 shows the UV-Visible spectra of branched metal nanostructures coupled to IgG according to an embodiment of the present invention.

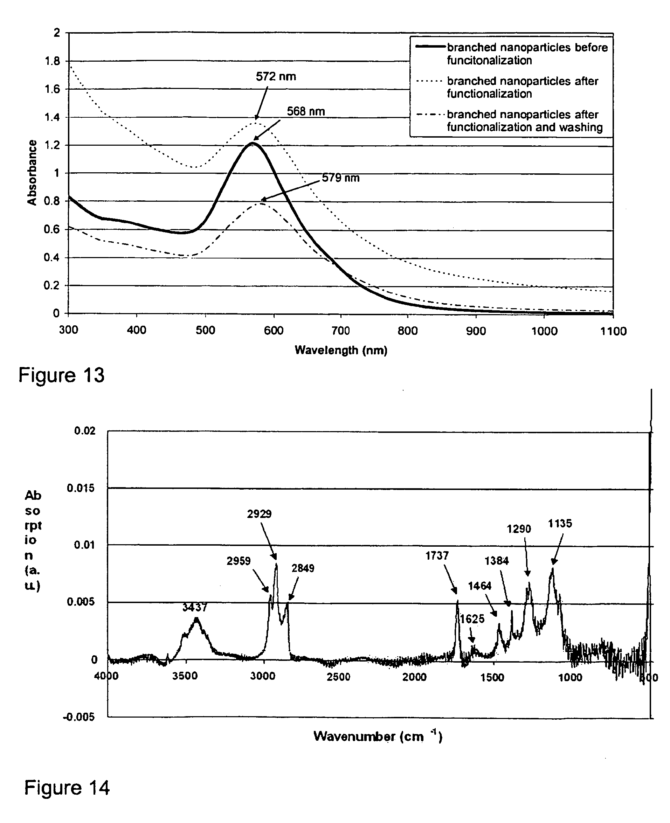

Figure 13 shows the UV-Visible spectra of branched metal nanostructures according to an embodiment of the present invention.
Figure 14 is the FTIR spectrum of metal nanostructures according to an embodiment of the present invention.

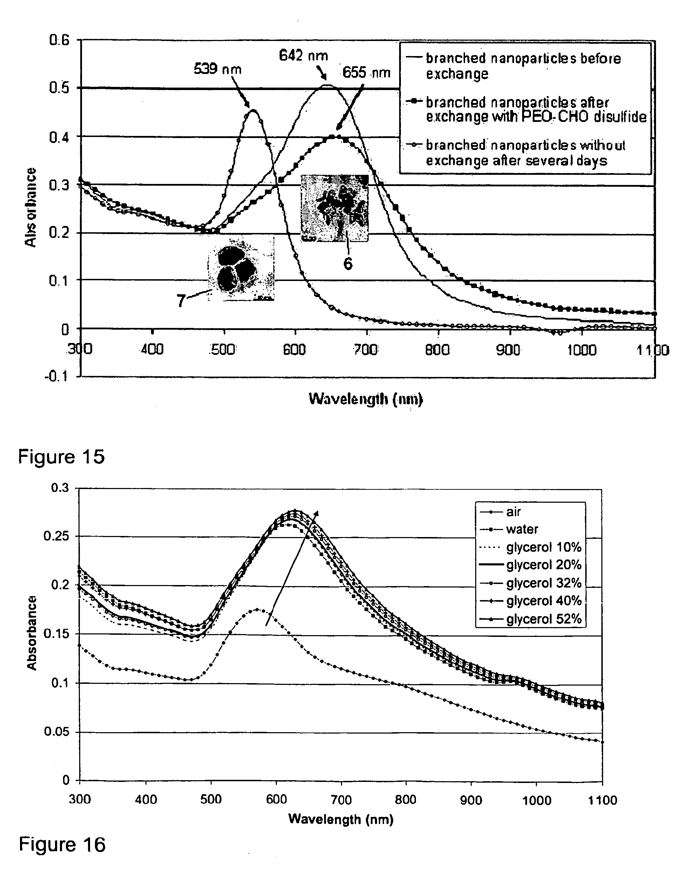

Figure 15 is an absorption spectrum showing the improved stability of metal nanostructures according to an embodiment of the present invention.
Figure 16 is an absorption spectra showing the sensibility of metal nanostructure films toward the refractive index of the surrounding media according to an embodiment of the present invention.

### Detailed description of the invention

In a first embodiment, the present invention relates to metal nanostructures. More precisely, these metal nanostructures are composed of a nanometric metal core having organic molecules linked at the surface thereof. Preferably, these molecules form a monolayer at the surface of the nanometric metal core. The nanometric metal core comprises gold and/or silver which can be optionally assembled together, alloyed or form an assembly or alloy with one or more other metals in proportions well known to the skilled person.

The nanometric metal cores are preferably particles having each dimension from 1 to 100 nm. Preferably, at least one dimension of said particles is from 10 to 90 nm, more preferably 20 and 75 nm. The metal nanostructures can have any shape such as, but not limited to, a sphere shape, a rod shape, a disk shape, a branched shape, a cuboidal shape or a pyramidal shape. Branched nanostructures are nanoparticles having one or more tips extending toward the outside of the particle.

The molecules attached to the nanometric metal core have the general formula W-X-Y-Z, wherein W is an atom selected from sulfur and seleniumbound to the nanometric metal core, X is a hydrophobic spacer, Y is a hydrophilic spacer and Z is either hydrogen or a is selected from the group consisting of amines, thiols, carboxylic acid halides, carboxylic acid anhydrides, isocyanates, isothiocyanates, sulfonyl halides, aldehydes, imines, epoxides, ketones, phosphoric esters, alcohols and the following species:

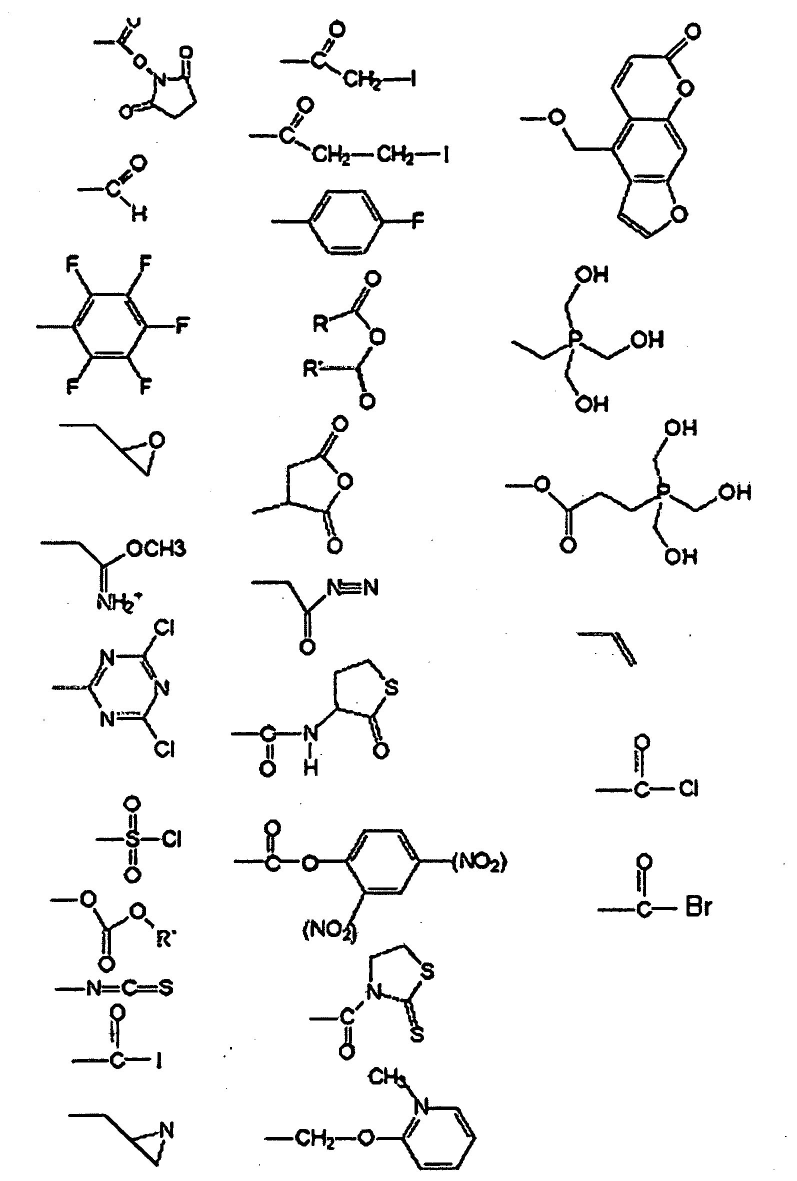

Suitable X hydrophobic spacers include, but are not limited to, hydrocarbon chains of the formula -(CH₂)ₙ- wherein n is from 8 to 30, preferably 9 and 30. X is important to realise stable self-assembled monolayers and therefore stable metal nanostructures. Preferably, at least one molecule linked to the surface of the nanometric metal core has a Z being a reactive group. Alternatively, all molecules present at the surface of the nanometric metal core have a Z being a reactive group. Suitable atoms W comprise, atoms like sulfur or selenium.

Suitable Y hydrophilic spacers are spacers preferably allowing solubility of the molecules in water-based solutions. Preferably, the Y spacer also avoids non-specific adsorption to biological compounds, cells or tissues. For this purpose, Y should preferably contains hydrogen-bond acceptors (e.g. fluorine, oxygen or nitrogen atoms possessing a lone electron pair), should preferably not contain hydrogen bond donors (atoms of fluorine, oxygen or nitrogen covalently bond to a hydrogen atom) and should preferably be overall electrically neutral. Preferably, the Y hydrophilic spacer is selected from the group consisting of polyethylene glycol, monosaccharides, oligosaccharides, polysaccharides, N-acetylpiperazine oligo(sarcosine) and permethylated sorbitol. Alternatively, the Y spacer can be made of two parts, e.g. a first part increasing the solubility in water (e.g. an ether, a crown ether, an amine, an amonium selt, an amide, an amino acid, a peptide, a sugar, a nitrile, a phosphonium salt or a phosphate) and a second part avoiding non-specific adsorption (e.g. polyethylene glycol). Due to the hydrophobic nature of the X compound (which is necessary for stability), Y is necessary to allow water-solubility of the molecules.

The metal nanostructures of the present invention are preferably water-suspendible under conventional conditions of concentration and/or temperature. Their water suspendibility is conferred by the Y spacers.

In another embodiment, the present invention relates to a process for preparing the metal nanostructures described herein. In an embodiment this process is performed by contacting a nanometric metal core such as defined above with one or more organic molecules having the general formula W'-X-Y-Z or Z-Y-X-W-W-X-Y-Z. By this process, a self-assembled monolayer of these one or more organic molecules is formed around the nanometric metal core and the particular morphology of the nanometric metal core is not changed. Additionally, the metal nanostructures are stabilised by this process. W, X, Y and Z are as defined above and W' is an atom or a chemical group able to bind the nanometric metal cores. Preferably, W' is a chemical group selected from thiol, selenol, sulfide of the general formula -S-R and selenide of the general formula -Se-R wherein R is selected from the group consisting of hydrogen and C₁-C₃₀ alkyl chains. Suitable X hydrophobic spacers comprise, but are not limited to, hydrocarbon chains having from 8 to 30 carbon atoms, preferably from 9 to 30 carbon atoms.

In a preferred embodiment, the nanometric metal cores are produced in such a way as to obtain nanometric metal cores of controlled shape. In this embodiment, the nanometric metal cores are grown in the presence of one or more capping agents. Typical capping agents include, but are not limited to, surfactants. Examples of suitable capping agents include, but are not limited to surfactants of the general formula CH₃(CH₂)ₙN⁺(CH₃)₃ X⁻ (n = integer of 1 to 15 and X = Br or Cl) (e.g. hexadecyltrimethylammonium bromide (CTAB)) or of the general formula (CH₃ (CH₂)ₙ)₄N⁺ X⁻ (n = integer of 1 to 15 and X = Br or Cl), benzyldimethylammonium chloride (BDAC), bis(p-sulfonatophenyl) phenylphosphine (BSPP), bis(2-ethylhexyl)sulfosuccinate (AOT), octylamine, dimyristoyl-*L*-α-phosphatidyl-*DL*-glycerol (DMPG), sodium dodecylsulfonate (SDS), ascorbic acid, sodium citrate and the like. In a preferred embodiment, the process is water-based, i.e. the nanoparticles are suspended in an aqueous solution. Another process for preparing the metal nanostructures of the present invention consists in contacting nanometric metal cores capped with capping agents as defined above with one or more molecules having the general formula W'-X-Y-Z or Z-Y-X-W-W-X-Y-Z as defined above and thereby exchanging the capping agents for the one or more molecules of the general formula W-X-Y-Z. as defined above.

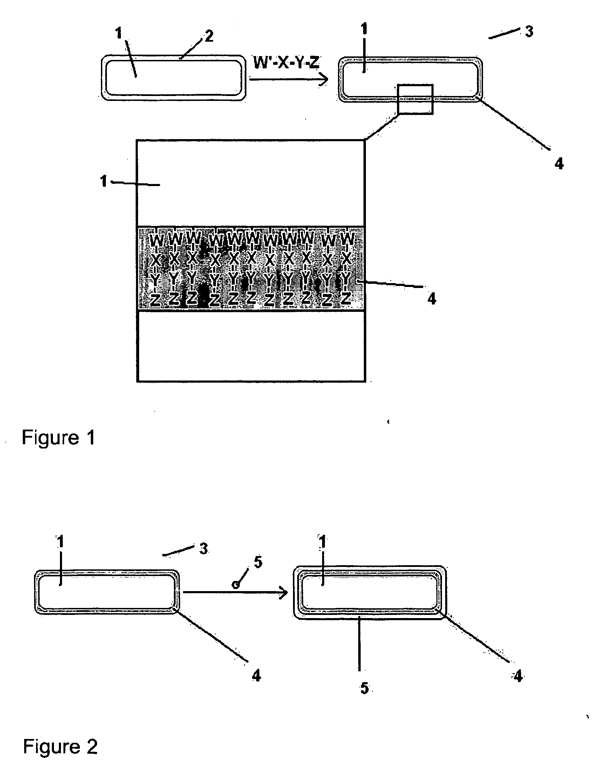

Figure 1 schematically shows the transformation of a nanometric metal core (1) surrounded by capping agents (2) into a metal nanostructure (3) according to the present invention via the exchange of the capping agent (2) by a self assembled monolayer (4) of molecules having the general formula W-X-Y-Z.

In another embodiment of the present invention, the metal nanostructures of the present invention are linked to one or more biomolecules via reaction or interaction of at least one reactive group Z with said one or more biomolecules. In a preferred but non-limiting embodiment, the biomolecules may be anti-bodies, antibody fragments or other receptors targeting cancer cells. This is schematically represented in Figure 2 showing the attachment of the nanostructures (3) of the present invention to biomolecules (5).

In another embodiment, the metal nanostructures of the present invention may be used to treat cancer by thermotherapy. The metal nanostructures of the present invention absorb near infra-red (hereinafter referred as NIR) light, i.e. light with a wavelength comprised between about 750 and about 1400 nm. Upon irradiation, the metal nanostructures of this invention generate heat which can be used to kill cancer cells. Ideally the metal nanostructures should:
- be sufficiently small to penetrate through the cancer cells,
- produce enough heat to kill the cells,
- be functionalized with antibodies, antibody fragments or other biomolecules targeting cancer cells, and
- be stable upon heating and in function of time.

All these desired properties are achieved using the metal nanostructures of the present invention.

In a first step, the method of treatment consist in either injecting the metal nanostructures directly into the tumour or in the bloodstream of the patient having a cancer tumour. In this last case, the metal nanostructure shall be linked to a biomolecule targeting cancer cells as described above. In a second step, the metal nanostructures now present in the tumour region are subjected to infrared irradiation. The resulting local increase in temperature causes the death of the tumour cells. In another embodiment, the metal nanostructures of the present invention are included in a pharmaceutical composition or are used in the manufacture of a pharmaceutical composition. This pharmaceutical composition is active against cancer under infrared irradiation.

In another embodiment the present invention relates to a film consisting of metal nanostructures according to the present invention. The film can be obtained by coating a substrate with a suspension of the metal nanostructures of the present invention. The method of coating can be any method known in the art such as but not limited to spraying, curtain coating, roller coating, doctor blade coating, dip coating, spin-coating, screen printing and the likes. The substrate can be any substrate but if the film is meant to be used in localised surface plasmon resonance sensors, the substrate is preferably transparent such as but not limited to PMMA, PS, glass or quartz. Quartz is the most preferred substrate. Preferably, the substrate is silanized in order to improve adherence of the film on the substrate. Metal nanostructures change their absorption behaviour upon differences in the refractive index at their surfaces. This property can be applied to sensing binding events like in the case of localised surface plasmon resonance sensors.

An illustration of how the film of the present invention can be used in a localised surface plasmon resonance sensor is given in example 4 below.

The nanostructures of the present invention can be used in sandwich assays. A sandwich assay is the base of one of the currently most applied diagnostic method, namely the ELISA test. In such an assay, an antibody probe is firstly immobilized onto a solid support, next this antibody probe can bind the antigen target. In a classical ELISA this binding event is visualised by contacting this complex with a secondary antibody which contains a certain label. These labels are typically absorption molecules, fluorescence labels or enzymatic labels. However, sometimes also metal nanostructures (e.g. Au or Ag) can be used to enhance the signal or can be used in combination with (optical) biosensor techniques. The extraordinary optical properties of non-spherical nanostructures, make them ideal as optical labels. However, the stability and the functionalization are key issues to use these nanostructures for this sort of applications. Due to their stability, the metal nanostructures of the present invention are therefore good candidates for use in sandwich arrays.

We now discuss in some details how nanometric metal cores of specific shape and composition, capped with capping agents, can be synthesised. Next, we will give working examples of how such nanometric metal cores of specific shape and compositions, capped with capping agents, can be stabilised according to various embodiments of the present invention by exchanging these capping agents with one or more molecules of the general formula W-X-Y-Z.

### Branched nanometric gold cores

The synthesis of branched nanometric gold cores may be based on a procedure known from Hao et al. (cited *supra*) Sodium citrate and bis(p-sulfonatophenyl)phenylphosphine dihydrate dipotassium (BSPP) were mixed and H₂O₂ was added. Next, under constant shaking 0.05M HAuCl₄ was added slowly at room temperature. Over several minutes, the solution color changed from colorless to blue. The resulting blue suspension can be kept in the refridgerator for several days, although eventually a spectral shift to the blue takes place. The nanocrystals loosed their branched morphology and relax into spherical particles. The amount and the sharpness of these branches is influenced by several parameters: the addition rate of HAuCl₄, the age of the mixture of citrate and BSPP, the concentration of HAuCl₄, the amount of BSPP and the amount of H₂O₂.

### Addition rate of HAuCl₄

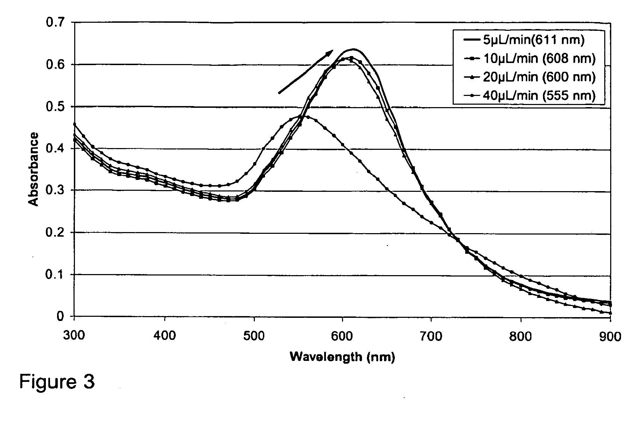

HAuCl₄ is the precursor salt that is reduced to form colloidal gold. This salt must be added slowly so the branches can grow in certain directions. The addition rate was varied from 5 to 40 µL per minute. The slower HAuCl₄ was added, the more red-shifted was the absorption maximum (Figure 3).

Figure 3 shows the UV-Vis absorption spectrum of the addition rate of HAuCl₄. The solution colour changes from red to purple to blue when going from faster to slower addition rate respectively.

### Age of the mixture of citrate and BSPP

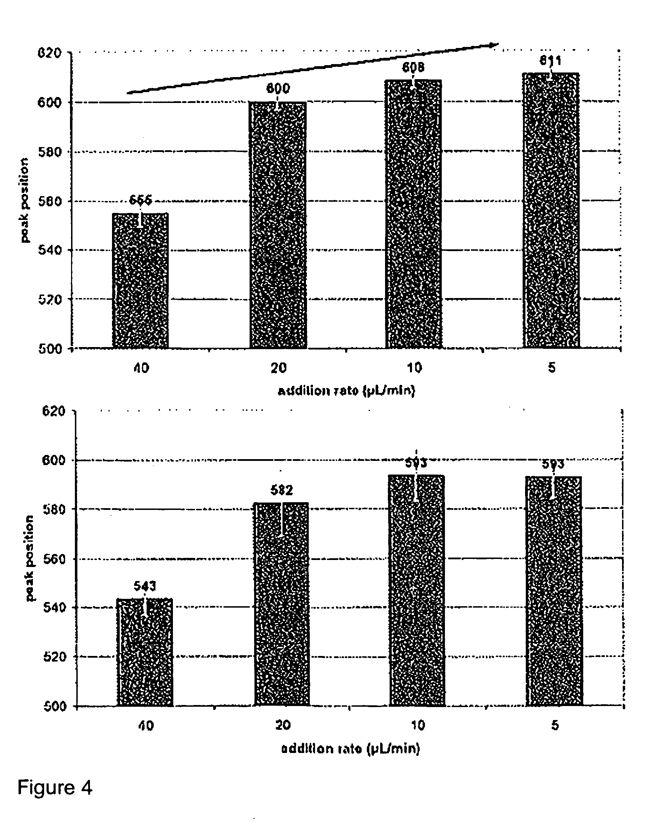

Citrate is a reducing agent for the reduction of HAuCl₄ into colloidal gold. It is also the capping agent that stabilizes the nanometric metal core in the beginning. BSPP is a detergent necessary to induce the growth in certain directions resulting in the branched morphology. Unfortunately, the shape only remains stable for a few days. Then the nanometric metal cores relax into a spherical morphology. This mixture of citrate and BSPP should be made freshly. In this way the branched nanometric metal cores show the best characteristics; the UV-Vis absorption maximum was shifted to higher wavelengths. When using a mixture that is one week old, the UV-Vis absorption maximum was shifted to lower wavelengths (Figure 4).

Figure 4 compares band positions of absorption maxima of branched nanostructures synthesized with a freshly prepared mixture (left) and synthesized with a mixture of one week old (right).

### The concentration of HAuCl₄

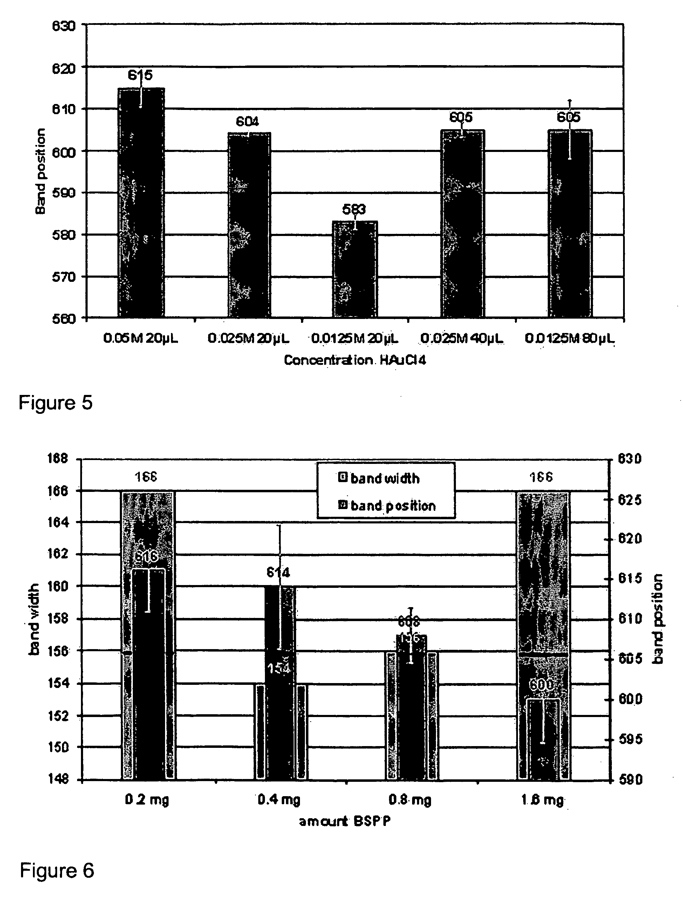

The concentration of HAuCl₄ was varied from 0.05M to 0.0125M for an addition rate of 10 µL/min. Firstly, the same amount (20µL) of HAuCl₄ for the different concentrations was taken. Secondly, the amount (40-80µL) was increased when decreasing the concentration (0.025-0.0125M). The best results were obtained with 20µL of 0.05M HAuCl₄ (Figure 5).

Figure 5 shows the band positions of absorption maxima for branched nanostructures synthesized with different concentrations of HAuCl₄.

### The amount of BSPP

BSPP is a detergent necessary to induce the growth in certain directions resulting in the branched morphology. The amount is varied from 0.2 to 1.6 mg in 10 mL of citrate solution. The best result concerning the position of the absorption maximum was obtained with 0.2 mg of BSPP (Figure 6).

Figure 6 shows the band positions of absorption maxima and band widths for branched nanometric metal cores synthesized with different amounts of BSPP.

### The amount of H₂O₂

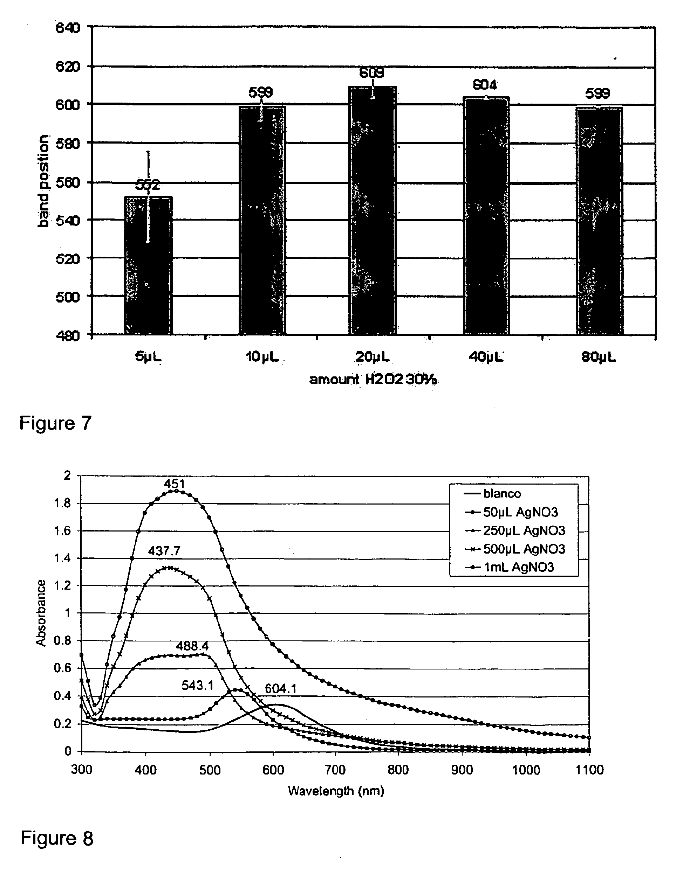

H₂O₂ is a reducing agent that is able to reduce HAuCl₄ together with citrate at room temperature. The amount of H₂O₂ was varied from 5 to 80 µL in 10 mL of citrate/BSPP solution. The best results were become with 20 µL of H₂O₂ (Figure 7).

Figure 7 shows band positions of absorption maxima of branched nanometric metal cores synthesized with different amounts of H₂O₂.

### Branched nanometric gold cores plated with silver

Branched nanometric gold cores were plated with silver in solution. In a procedure, 2.5 mL of branched nanometric gold cores were mixed with 1.5 mL of Ascorbic Acid 10⁻² M (reducing agent). Then 20 mL of H₂O was added under constant shaking. Finally, different amounts of AgNO₃ 10⁻² M were added going from 0 µL (blanco) to 1 mL of AgNO₃. Upon addition of AgNO₃ the absorption maximum of the gold shifts to lower wavelength and disappears slowly when a new absorption maximum of silver appears (Figure 8).

Figure 8 shows the UV-Vis absorption spectra of branched nanometric gold cores plated with silver in solution.

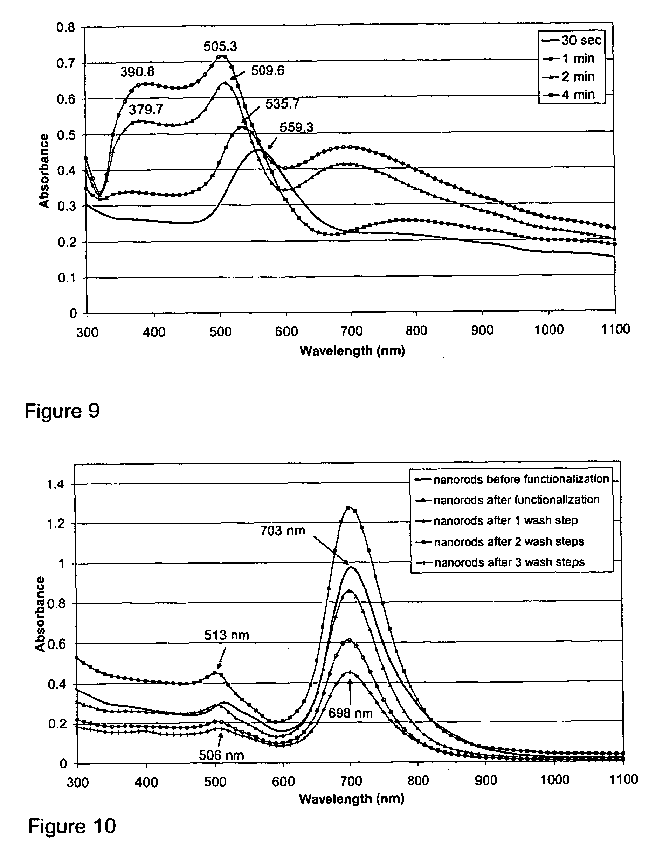

Branched nanometric gold cores were also plated with silver on a surface. Therefore, the branched nanometric gold cores were deposited on a silanized quartz substrate. Then these substrates were held in a 1/1 mixture of AgNO₃ and hydroquinone for different periods of time going from 30 seconds to 4 minutes. For short periods of time the absorption maximum shift to lower wavelengths. For longer plating times (2 and 4 minutes) a second absorption maximum appears around 400 nm because of the larger amount of plated silver (Figure 9).

Figure 9 shows the UV-Vis absorption spectra of branched nanometric gold cores plated with silver on a surface.

### Gold nanorods

Others have made nanocrystals with other morphologies like cubes, triangular nanoplats, pyramids, etc... One of these interesting morphologies are nanorods. The synthesis of these nanorods is described in for example Sau et al. (Langmuir 2004, 20(15), 6416), Nikoobakht et al. (Chemistry of Materials 2003, 15(10), 1957) and Jana et al. (The Journal of Physical Chemistry B 2001, 105(19), 4065). They all start with the synthesis of little gold nanoparticles (seeds) which they grow into nanorods in a second step.

### EXAMPLE 1 - preparation of a stabilised gold nanorods solution

0.250 ml of an aqueous 0.01 M solution of HAuCl₄.3H₂O was added to 7.5 ml of a 0.10 M CTAB solution in a test tube. The solution was gently mixed by inverting the tube repeatedly. The solution appeared bright brown-yellow in color. Then, 0.600 ml of an aqueous 0.01 M ice-cold NaBH₄ solution was added at once, followed by a rapid mixing (by inverting the tube) for 2 min. Car was taken to allow the escape of the evolved gas during mixing. The solution developed a pale brown-yellow color.

2 mL of the gold nanorods containing solution was brought to pH = 11 with NaOH. Next, the gold nanorods were mixed with 200 µL of 12 mM of S₂-C₁₁-PEO₄-CHO in water, which is an abbreviation for:

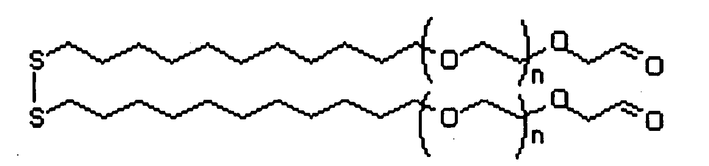

The function of this compound in to replace the original CTAB capping agents around the nanometric metal cores. This approach generated nanorods with aldehyde group functionalities. The aldehyde group functionalized gold nanorods were purified from the excess of S₂-C₁₁-PEO₄-CHO by ultracentrifugation of the gold nanorods. They were consequently washed with ultra-pure water. This procedure was repeated three times to ensure purified aldehyde group functionalised gold nanorods. In every purification step, some gold nanorods were lost but characteristic spectral properties of the gold nanorods were still visible. An alternative approach to purify the obtained nanostructures consists in performing a dialysis against ultrapure water.

Experimental evidence that the functionalisation was successful is shown in the UV-Vis absorption spectrum (Figure 3) and in the Fourier Transformed infra Red (FTIR) spectrum (in a KBr pellet) of the resulting gold nanorods (Figure 4).

Figure 3 shows the UV-visible absorption spectrum of gold nanorods before (plain line) and after the functionalization with S2-C11-PEO4-CHO molecules (squares) and after a first (triangles), a second (circles) and a third washing step (crosses). A shift in the peak position from 703 nm to 698 nm is a first indication that the functionalisation was successful. The attributions of the bands visible in Figure 4 are detailed in Table 1 below.

**Table 1**

| Band (cm⁻¹) | Description |
|---|---|
| 3437 | Bounded H₂O |
| 2961 | ν (CH₂) near aldehyde |
| 2928 and 2849 | ν asymm and symm CH₂ |
| 1734 | ν (C=O) |
| 1633 | δ (OH) of H₂O |
| 1465 and 1384 | δ asymm and symm (CH₂) |
| 1280 and 1127 | ν asymm and symm (C-O-C) |

The morphology of the gold nanorods did not change during this functionalization step, which was verified by comparing TEM images before and after functionalization and purification of the gold nanorods.

The stability of these metal nanostructures was excellent over a long period of time (the shape and the spectral properties of these gold nanorods remained unchanged in months), which shows the strength and advantages of this approach.

### EXAMPLE 2 - coupling of a gold nanorod with a biomolecule

The functionalized gold nanorods of example 1 were covalently coupled to antibodies. For this purpose, 10 µL of the functionalized gold nanorods solution was mixed with 100 µL of 2 mg/mL IgG. This IgG solution was prepared by mixing 50 µL IgG of 6 mg/mL with 100 µL cyanoborohydride buffer (which was brought to pH = 5). After coupling of the IgG to the functionalized gold nanorods, the nanorods were purified via ultracentrifuge and washed with phosphate buffer. The existence of the coupling was verified by the observation of a shift from 698 nm to 708 nm in the peak position in the resulting absorption spectrum (see Figure 4).

Figure 4 shows the UV-visible spectrum of the functionalized gold nanorods before (circles) and after IgG coupling by using a ratio nanorod: IgG of 1:10 (plain line) or 1:1 (squares).

### EXAMPLE 3 - preparation of stabilised branched gold nanostructures

100 ml of a 6.8 x 10-3 M sodium citrate solution and 4 mg bis(p-sulfonatophenyl)phenylphosphine dihydrate dipotassium (BSPP) were mixed and 0.2 ml of 30% H₂O₂ was added. Next, under constant shaking 200 µl of 0.05M HAuCl₄ was added slowly at room temperature. Over several minutes, the solution colour changed from colourless to blue.

2 mL of this solution was brought to pH = 11 with NaOH. Next, the nanorods were mixed with 200 µL of 12 mM of S₂-C₁₁-PEO₄-CHO in water. The purpose of this compound is to replace the original BSPP/citrate capping around the nanometric metal cores. This approach generates branched metal nanostructures with aldehyde group functionality. The aldehyde group functionalized branched metal nanostructures are purified from the excess of S₂-C₁₁-PEO₄-CHO by ultracentrifugation of the branched metal nanostructures to the bottom of the vial and wash them consequently with ultra-pure water. This procedure was repeated three times to ensure purified aldehyde group functionalized branched metal nanostructures. By every purification step, some branched metal nanostructures are lost but characteristic spectral properties of the branched metal nanostructures are still visible (see figure 6). An alternative approach to purify the obtained metal nanostructures consists in performing a dialysis against ultra-pure water.

Figure 6 shows the UV-visible absorption spectrum of branched nanostructures before (plain line), after the functionalisation with S2-C11-PEO4-CHO molecules (dotted line), and after the purification of these branched metal nanostructures (dash-dot line). A shift in the peak position from 568 nm to red-shifted wavelength is a first indication that the functionalisation was successful.

An experimental evidence that the functionalisation was successful is shown in the Fourier Transformed infra Red (FTIR) spectrum (in a KBr pellet) of the resulting gold nanorods (Figure 7).

The morphology of the branched metal nanostructures did not change during this functionalization step, which was verified by comparing TEM images before and after functionalization and purification of the gold nanorods.

The stability of these metal nanostructures was excellent over a long period of time (months), which shows the strength and advantages of this approach. Not coated nanometric metal cores changed to spherical nanometric metal cores in a period of 2 to 3 days.

Figure 8 shows the UV-Visible spectrum of branched nanometric gold cores before (plain line) and 3 days after exchange (squares) with S2-C11-PEO4-CHO. Insert (6) in Figure 8 shows a TEM picture of a gold nanostructure corresponding to the square line. It is clear from this picture that the gold nanostructures still kept their branched shape 3 days after synthesis and functionalisation. The absorption spectrum of unfunctionalized branched nanometric gold cores 3 days after synthesis are presented for comparison purpose (circles). Insert (7) in Figure 8 shows a TEM picture of a nanometric gold core corresponding to the circle line. It is clear from this picture that ,contrarily to the functionalized gold nanostructure, the unfunctionalized nanometric gold core came back to a spherical shape after three days. This change is accompanied by a large (>100 nm) blue shift.

### EXAMPLE 4 - sensitivity of a gold nanostructure film to dielectric environment

Metal nanostructure films (the metal used being specified in table 2 below) were obtained by deposition of metal nanostructures onto a silanized quartz substrate using metal nanostructures suspensions. Sensitivity measurements on these films were done by changing the dielectric environment of the metal nanostructures (different glycerol concentrations). Both the maximum absorbence and the wavelength at that position were monitored during variation of the refractive index (from 1.008 for air to 1.4011 for 52% glycerol).

Figure 9 shows the UV-Visible absoption spectrum of the metal nanostructure film. It shows the red-shift in wavelength and the increase in absorbance observed for increasing concentration of glycerol.

The relative shift in peak position, Δλmax, and the relative shift in absorbance, Δabs, are linearly dependent on the refractive index of the surrounding medium. From the slope of these plots, which could be obtained by linear regression, the sensitivity of the films (Δλmax/Δn or Δabs/Δn) was calculatedⁱ.

Table 2 below provides the slopes for the wavelength and absorbance curves.

**Table 2**

| | |
|---|---|
| Metal nanostructure film | Δλmax/Δn |
| Spherical gold (50 nm) | 64.79 |
| Branched gold | 144.02 |
| Branched gold + silver plating (on surface) | 197.09 (2 minutes plating) |
| Branched gold + silver plating (in solution) | 214.1 (250 µL AgNO3) |

Branched gold nanostructures with silver plating films give the highest sensitivity for changes in wavelength due to the change of refractive index. The spherical nanometric gold cores film give the lowest sensitivity for changes in wavelength due to change in refractive index.

## Claims

1. A metal nanostructure comprising:
- a nanometric metal core comprising gold, silver or an assembly or alloy of gold and silver, and
- one or more molecules attached to one or more surfaces of said nanometric metal core,
**characterised in that** each of said one or more molecules has the structural formula W-X-Y-Z, wherein W is an atom selected from sulfur and selenium bound to said nanometric metal core, X is a hydrophobic spacer, Y is a hydrophilic spacer and Z is hydrogen or is selected from the group consisting of amines, thiols, carboxylic acid halides, carboxylic acid anhydrides, isocyanates, isothiocyanates, sulfonyl halides, aldehydes, imines, epoxides, ketones, phosphoric esters, alcohols and the following species:

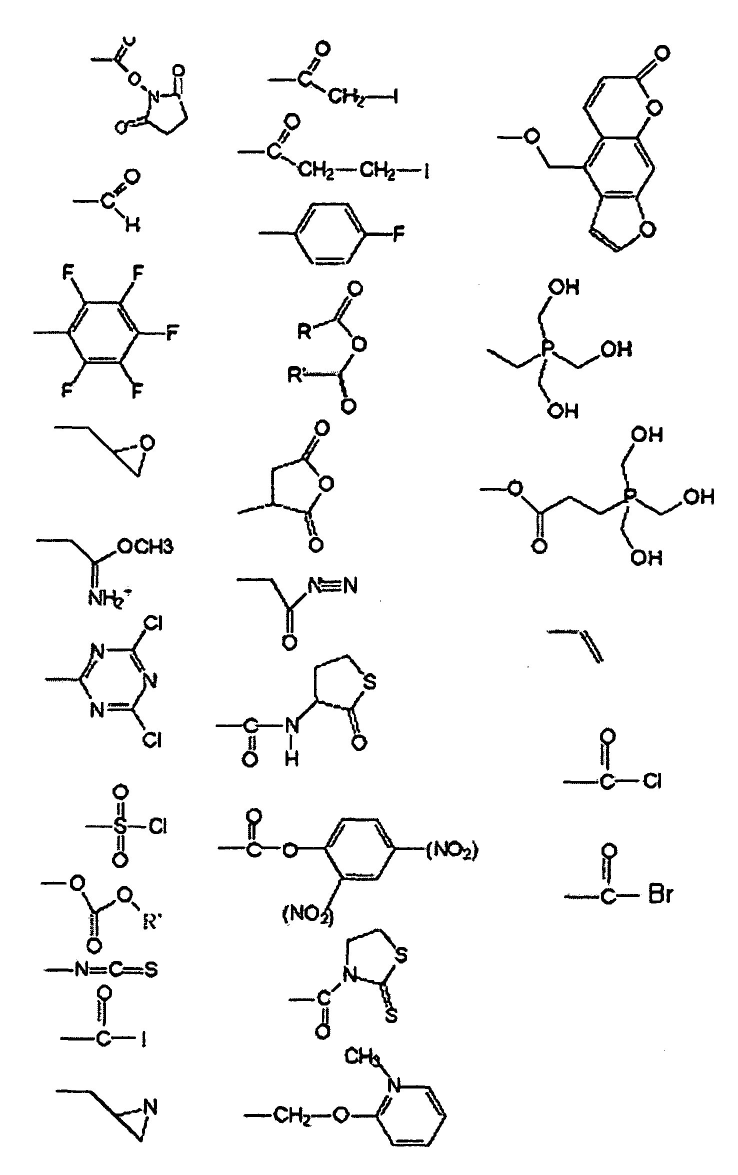

2. A metal nanostructure according to claim 1, **characterised in that** each dimension of said nanometric metal core is from 1 to 100 nm.

3. A metal nanostructure according to any of claims 1 and 2, **characterised in that** said metal nanostructure has a pyramidal or branched shape.

4. A metal nanostructure according to any of claims 1 to 3, **characterised in that** each said one or more molecules forms a monolayer at one or more surfaces of said nanometric metal core.

5. A metal nanostructure according to any of claims 1 to 4, **characterised in that** the hydrophobic spacer X is a hydrocarbon chain having from 8 to 30 carbon atoms.

6. A metal nanostructure according to any of claims 1 to 5, **characterised in that** Y is an electrically neutral hydrophilic spacer containing hydrogen-bond acceptors, but free from hydrogen bond donors.

7. A metal nanostructure according to any of claims 1 to 6, **characterised in that** Y is a polyethylene glycol, a monosaccharide, an oligosaccharide, a polysaccharide, a N-acetylpiperazine, an oligo(sarcosine) or a permethylated sorbitol group.

8. A process for preparing a metal nanostructure according to claim 1, **characterized in that** a nanometric metal core is contacted with one or more molecules having the structural formula W'-X-Y-Z or Z-Y-X-W-W-X-Y-Z, wherein each of W and W' is an atom selected from sulfur and selenium, X is a hydrophobic spacer, Y is a hydrophilic spacer and Z is hydrogen or is selected from the group consisting of amines, thiols, carboxylic acid halides, carboxylic acid anhydrides, isocyanates, isothiocyanates, sulfonyl halides, aldehydes, imines, epoxides, ketones, phosphoric esters, alcohols and the following species:

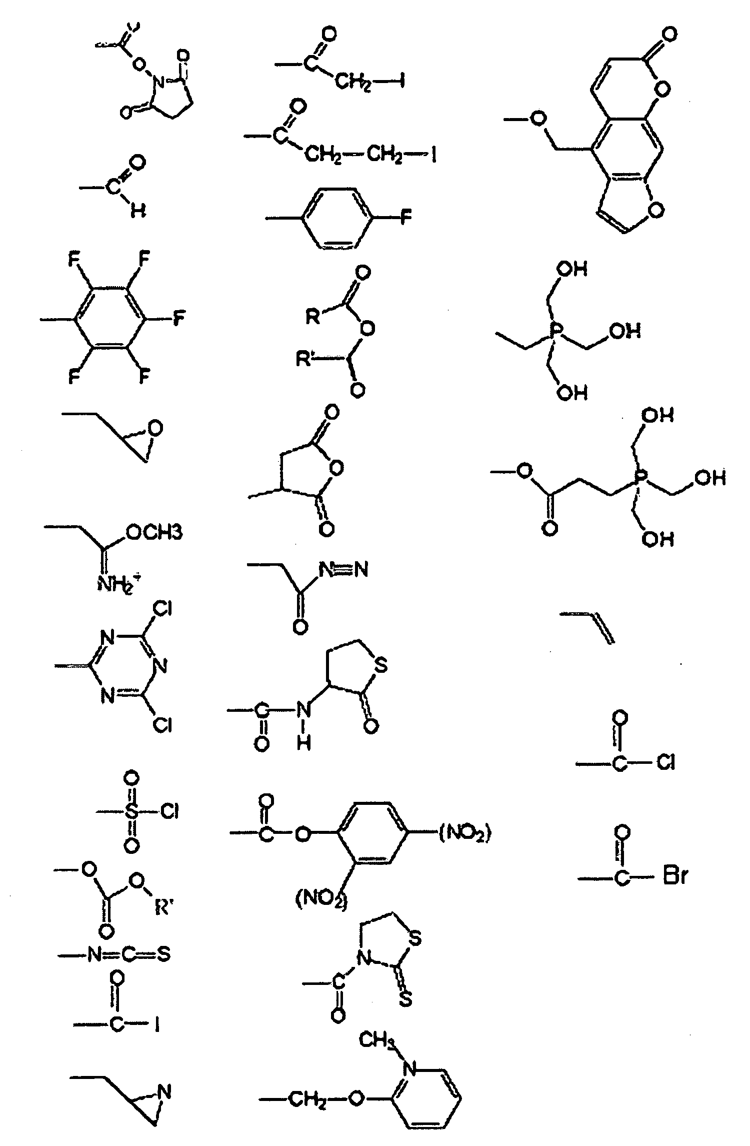

9. A process according to claim 8, **characterised in that** one or more capping agents are attached to said nanometric metal core and **in that** said preparation operates by exchanging said one or more capping agents by said one or more molecules having the general formula W'-X-Y-Z or Z-Y-X-W-W-X-Y-Z.

10. A process according to claim 9, **characterised in that** said capping agents are selected from the group consisting of CH₃(CH₂)ₙN⁺(CH₃)₃ X⁻ or (CH₃ (CH₂)ₙ)₄N⁺ X⁻ , wherein n = integer of 1 to 15 and X = Br or Cl, benzyldimethylammonium chloride, bis(p-sulfonatophenyl)phenylphosphine, bis(2-ethylhexyl)sulfosuccinate , octylamine, dimyristoyl-*L*-α-phosphatidyl-DL-glycerol, sodium dodecylsulfonate, ascorbic acid and sodium citrate.

11. A process according to claim 9 or claim 10, **characterised in that** W' is a chemical group selected from R-S- and R-Se-, wherein R is selected from the group consisting of hydrogen and C₁-C₃₀ alkyl chains, and W is an atom selected from sulphur and selenium.

12. A film supported on a substrate, said film comprising a metal nanostructure according to any of claims 1 to 7.

13. A process for making a film according to claim 12, comprising the steps of:
(i) suspending a metal nanostructure according to any of claims 1 to 7 in an aqueous medium, and
(i) depositing said suspension onto a substrate.

14. The use of a film according to claim 12 for measuring a change of refractive index.

15. A pharmaceutical composition including a metal nanostructure according to any of claims 1 to 7 and optionally one or more pharmaceutically acceptable excipients.

16. A pharmaceutical composition according to claim 15, wherein the proportion of said metal nanostructure in said composition is from 0.01 to 99.99 % by weight.

17. A pharmaceutical composition according to claim 15 or 16, being active under infrared irradiation.

## Patentansprüche

1. Metall-Nanostruktur, umfassend:
- einen nanometrischen Metallkern, der Gold, Silber oder einen Verbund oder eine Legierung von Gold und Silber aufweist, und
- ein oder mehrere Moleküle, die an ein oder mehrere Oberflächen des nanometrischen Metallkerns anhaften,
**dadurch gekennzeichnet, dass** jedes der ein oder mehreren Moleküle die Strukturformel W-X-Y-Z aufweist, wobei W ein Atom ist, ausgewählt aus Schwefel und Selen, gebunden an den nanometrischen Metallkern, X ein hydrophober Spacer ist, Y ein hydrophiler Spacer ist und Z Wasserstoff ist oder ausgewählt ist aus der Gruppe, bestehend aus Aminen, Thiolen, Carbonsäurehalogeniden, Carbonsäureanhydriden, Isocyanaten, Isothiocyanaten, Sulfonylhalogeniden, Aldehyden, Iminen, Epoxiden, Ketonen, Phosphorsäureestern, Alkoholen und den nachfolgenden Species:

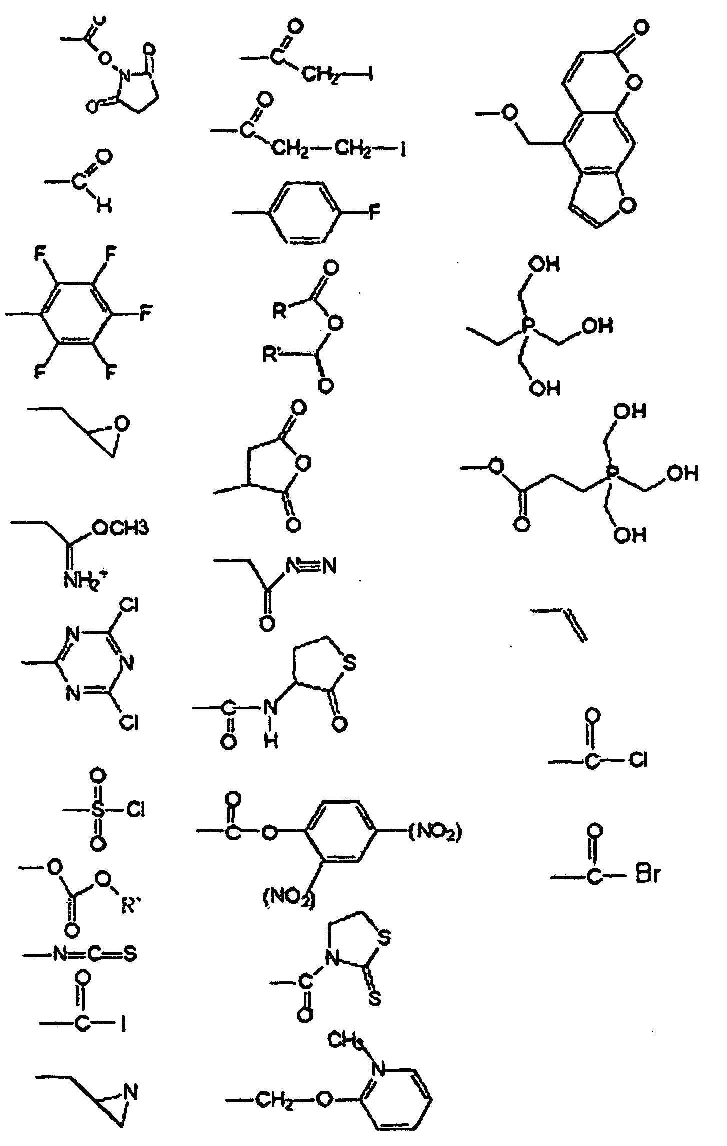

2. Metall-Nanostruktur nach Anspruch 1, **dadurch gekennzeichnet, dass** jede Dimension des nanometrischen Metallkerns 1 bis 100 nm beträgt.

3. Metall-Nanostruktur nach einem der Ansprüche 1 und 2, **dadurch gekennzeichnet, dass** die Metall-Nanostruktur eine pyramidale oder verzweigte Form besitzt.

4. Metall-Nanostruktur nach einem der Ansprüche 1 bis 3, **dadurch gekennzeichnet, dass** jedes der ein oder mehreren Moleküle eine Monoschicht an ein oder mehreren Oberflächen des nanometrischen Metallkerns ausbildet.

5. Metallische Nanostruktur nach einem der Ansprüche 1 bis 4, **dadurch gekennzeichnet, dass** der hydrophobe Spacer X eine Kohlenwasserstoffkette mit 8 bis 30 Kohlenstoffatomen ist.

6. Metall-Nanostruktur nach einem der Ansprüche 1 bis 5, **dadurch gekennzeichnet, dass** Y ein elektrisch neutraler hydrophiler Spacer ist, der wasserstoffgebundene Akzeptoren enthält, jedoch keine wasserstoffgebundene Donoren.

7. Metall-Nanostruktur nach einem der Ansprüche 1 bis 6, **dadurch gekennzeichnet, dass** Y ein Polyethylenglycol, ein Monosaccharid, ein Oligosaccharid, ein Polysaccharid, ein N-Acetylpiperazin, ein Oligo(sarcosin) oder eine permethylierte Sorbitolgruppe ist.

8. Verfahren zur Herstellung einer Metall-Nanostruktur nach Anspruch 1, **dadurch gekennzeichnet, dass** ein nanometrischer Metallkern mit ein oder mehreren Molekülen mit der Strukturformel W'-X-Y-Z oder Z-Y-X-W-W-X-Y-Z in Kontakt gebracht wird, wobei jedes W und W' ein Atom ist, ausgewählt aus Schwefel und Selen, X ein hydrophober Spacer ist, und Y ein hydrophiler Spacer ist und Z Wasserstoff ist oder ausgewählt ist aus der Gruppe, bestehend aus Aminen, Thiolen, Carbonsäurehalogeniden, Carbonsäureanhydriden, Isocyanaten, Isothiocyanaten, Sulfonylhalogeniden, Aldehyden, Iminen, Epoxiden, Ketonen, Phosphorsäureestern, Alkoholen und den nachfolgenden Species:

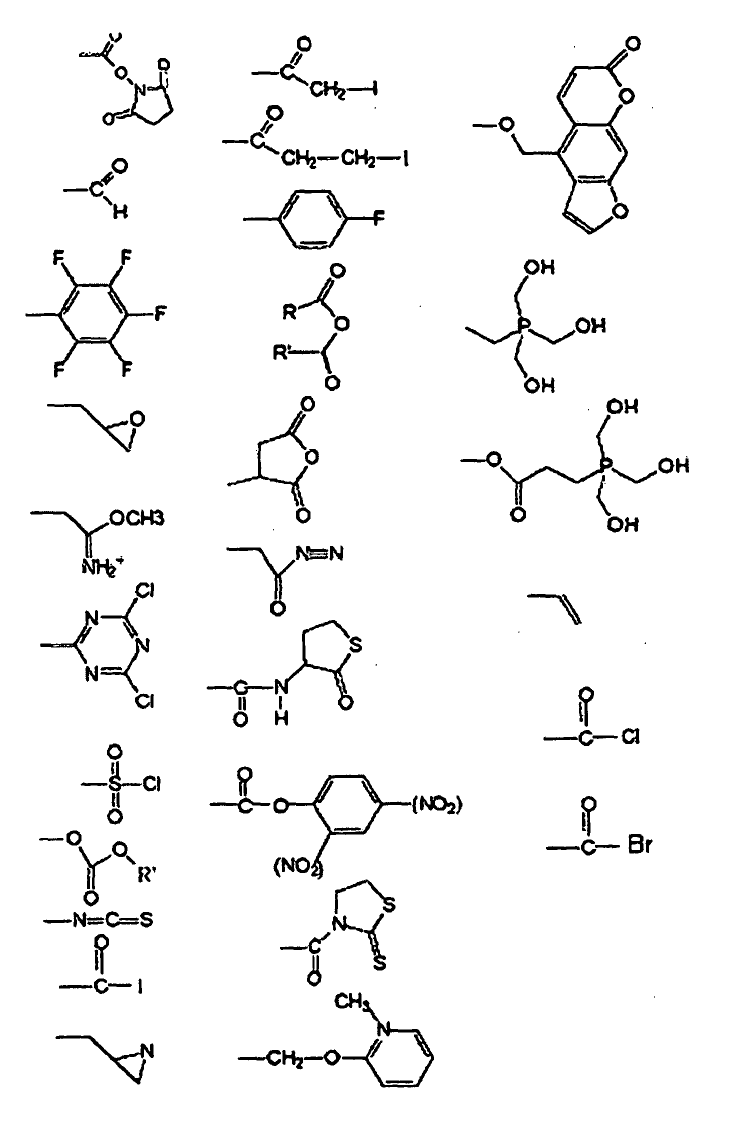

9. Verfahren nach Anspruch 8, **dadurch gekennzeichnet, dass** ein oder mehrere Schutzmittel an den nanometrischen Metallkern angeheftet sind und dass die Herstellung durch Austausch der ein oder mehreren Schutzmittel durch ein oder mehrere Moleküle mit der allgemeinen Formel W'-X-Y-Z oder Z-Y-X-W-W-X-Y-Z stattfindet.

10. Verfahren nach Anspruch 9, **dadurch gekennzeichnet, dass** die Schutzmittel ausgewählt werden aus der Gruppe, bestehend aus CH₃ (CH₂)ₙN⁺(CH₃)₃ X⁻ oder (CH₃(CH₂)ₙ)₄N⁺ X⁻, wobei n = Ganzzahliges von 1 bis 15 und X = Br oder Cl, Benzyldimethylammoniumchlorid, Bis(p-sulfonatophenyl)phenyl-phosphin, Bis(2-ethylhexyl)sulfosuccinat, Octylamin, Dimyristoyl-L-α-phosphatidyl-DL-glycerol, Natriumdodecylsulfonat, Ascorbinsäure und Natriumcitrat.

11. Verfahren nach Anspruch 9 oder Anspruch 10, **dadurch gekennzeichnet, dass** W' eine chemische Gruppe ist, ausgewählt aus R-S- und R-Se-, wobei R ausgewählt ist aus der Gruppe, bestehend aus Wasserstoff und C₁-C₃₀-Alkylketten, und W ein Atom ist, ausgewählt aus Schwefel und Selen.

12. Film, der auf einem Träger aufliegt, wobei der Film eine Metall-Nanostruktur nach einem der Ansprüche 1 bis 7 aufweist.

13. Verfahren zur Herstellung eines Films nach Anspruch 12, mit den Schritten:
(i) Suspendieren einer Metall-Nanostruktur nach einem der Ansprüche 1 bis 7 in einem wässrigen Medium, und
(ii) Ablagern der Suspension auf einem Träger.

14. Verwendung eines Films nach Anspruch 12 zur Bestimmung einer Änderung des Brechungsindex.

15. Pharmazeutische Zusammensetzung mit einer Metall-Nanostruktur nach einem der Ansprüche 1 bis 7 und wahlweise ein oder mehreren pharmazeutisch verträglichen Arzneimittelträgern.

16. Pharmazeutische Zusammensetzung nach Anspruch 15, wobei der Anteil der Metall-Nanostruktur in der Zusammensetzung 0,01 bis 99,99 Gew.-% beträgt.

17. Pharmazeutische Zusammensetzung nach Anspruch 15 oder 16, die bei Infrarotbestrahlung aktiv ist.

## Revendications

1. Nanostructure métallique comprenant :
- un noyau métallique nanométrique comprenant de l'or, de l'argent ou un assemblage ou un alliage d'or et d'argent, et
- une ou plusieurs molécules attachées à une ou plusieurs surfaces dudit noyau métallique nanométrique,
**caractérisée en ce que** chacune desdites une ou plusieurs molécules a la formule structurelle W-X-Y-Z, dans laquelle W est un atome sélectionné parmi le soufre et le sélénium lié audit noyau métallique nanométrique, X est un espaceur hydrophobe, Y est un espaceur hydrophile et Z est de l'hydrogène ou est sélectionné parmi le groupe constitué des amines, des thiols, des hydracides carboxyliques, des anhydrides acides carboxyliques, des isocyanates, des isothiocyanates, des halogénures de sulfonyl, des aldéhydes, des imines, des époxydes, des cétones, des esters phosphoriques, des alcools et des espèces suivantes :

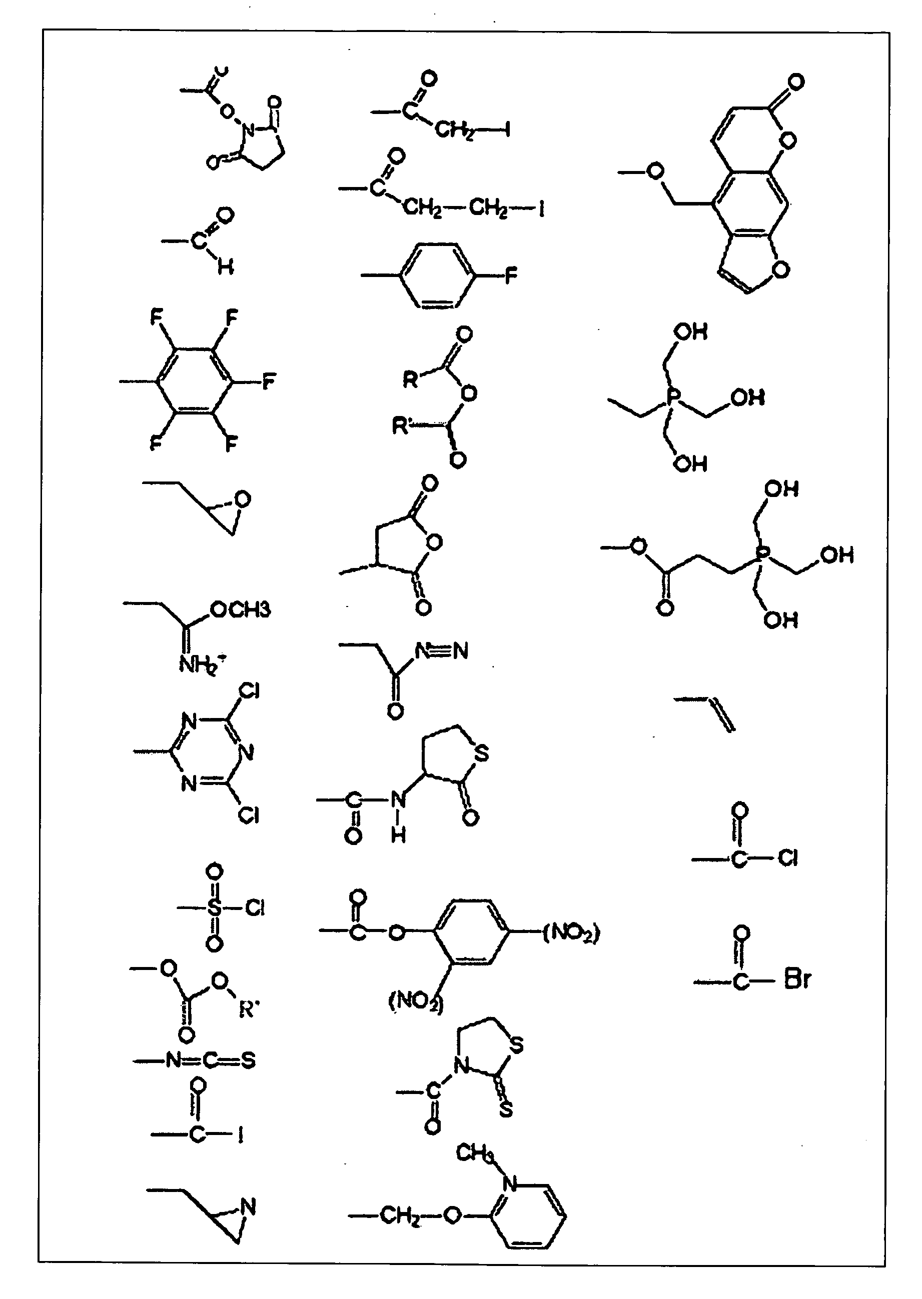

2. Nanostructure métallique selon la revendication 1, **caractérisée en ce que** chaque dimension dudit noyau métallique nanométrique est comprise entre 1 et 100 nm.

3. Nanostructure métallique selon l'une quelconque des revendications 1 et 2, **caractérisée en ce que** ladite nanostructure métallique a une forme pyramidale ou ramifiée.

4. Nanostructure métallique selon l'une quelconque des revendications 1 à 3, **caractérisée en ce que** chaque dite une ou plusieurs molécules forme une monocouche au niveau d'une ou plusieurs surfaces dudit noyau métallique nanométrique.

5. Nanostructure métallique selon l'une quelconque des revendications 1 à 4, **caractérisée en ce que** l'espaceur hydrophobe X est une chaîne hydrocarbonée ayant de 8 à 30 atomes de carbone.

6. Nanostructure métallique selon l'une quelconque des revendications 1 à 5, **caractérisée en ce que** Y est un espaceur hydrophile électriquement neutre contenant des accepteurs de liaison hydrogène, mais exempt de donneurs de liaison hydrogène.

7. Nanostructure métallique selon l'une quelconque des revendications 1 à 6, **caractérisée en ce que** Y est un glycol de polyéthylène, un monosaccharide, un oligosaccharide, un polysaccharide, un N-acétylpiperazine, un oligo(sarcosine) ou un groupe sorbitol perméthylaté.

8. Processus pour préparer une nanostructure métallique selon la revendication 1, **caractérisé en ce qu'**un noyau métallique nanométrique est mis en contact avec une ou plusieurs molécules ayant la formule structurelle W'-X-Y-Z ou Z-Y-X-W-W-X-Y-Z, dans laquelle chacun des W et W' est un atome sélectionné parmi le soufre et le sélénium, X est un espaceur hydrophobe, Y est un espaceur hydrophile et Z est de l'hydrogène ou est sélectionné parmi le groupe constitué des amines, des thiols, des hydracides carboxyliques, des anhydrides acides carboxyliques, des isocyanates, des isothiocyanates, des haloïdes de sulfonyl, des aldéhydes, des imines, des époxydes, des cétones, des esters phosphoriques, des alcools et des espèces suivantes :

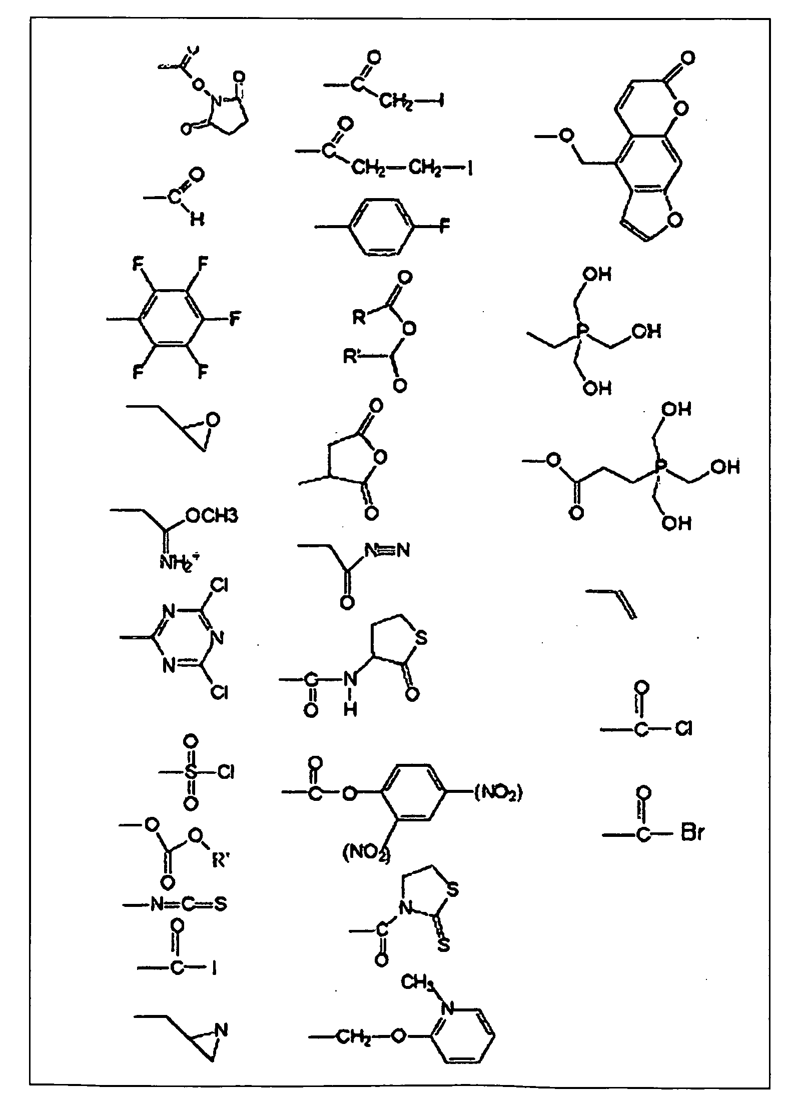

9. Processus selon la revendication 8, **caractérisé en ce qu'**un ou plusieurs agents de cioffage sont attachés audit noyau métallique nanométrique et **en ce que** ladite préparation fonctionne en échangeant lesdits un ou plusieurs agents de coiffage par ladite une ou plusieurs molécules ayant la formule générale W'-X-Y-Z ou Z-Y-X-W-W-X-Y-Z.

10. Processus selon la revendication 9, **caractérisé en ce que** lesdits agents de coiffage sont sélectionnés à partir du groupe constitué de CH₃ (CH₂)ₙN⁺ (CH³)₃ X⁻ ou (CH₃ (CH₂)ₙ)₄N⁺ X⁻, où n = entier de 1 à 15 et X = Br ou Cl, du chlorure de benzyldiméthylammonium, du bis(p-sulfonatophényl) phénylphosphine, du bis(2-éthylhexyle)sulfosuccinate, de l'octylamine, du dimyristoyl-*L*-α-phosphatidyle-*DL-*glycerol, du dodecylsulfonate de sodium, de l'acide ascorbique et du citrate de sodium.

11. Processus selon la revendication 9 ou la revendication 10, **caractérisé en ce que** W' est un groupe chimique sélectionné parmi R-S- et R-Se-, où R est sélectionné à partir du groupe constitué de l'hydrogène et des chaînes alkyles C₁ - C₃₀, et W est un atome sélectionné parmi le soufre et le sélénium.

12. Film supporté sur un substrat, ledit film comprenant une nanostructure métallique selon l'une quelconque des revendications 1 à 7.

13. Processus pour fabriquer un film selon la revendication 12, comprenant les étapes consistant à :
(i) mettre en suspension une nanostructure métallique selon l'une quelconque des revendications 1 à 7 dans un milieu aqueux, et
(i) déposer ladite suspension sur un substrat.

14. Utilisation d'un film selon la revendication 12 pour mesurer un changement d'indice de réfraction.

15. Composition pharmaceutique comprenant une nanostructure métallique selon l'une quelconque des revendications 1 à 7 et de manière facultative un ou plusieurs excipients acceptables d'un point de vue pharmaceutique.

16. Composition pharmaceutique selon la revendication 15, dans laquelle la proportion de ladite nanostructure métallique dans ladite composition est de 0,01 à 99,99 % en poids.

17. Composition pharmaceutique selon la revendication 15 ou 16, active sous un rayonnement infrarouge.
